# EUROPEAN PATENT SPECIFICATION

(11) **EP 1 228 052 B1**
(45) Date of publication and mention of the grant of the patent: **02.05.2007**
(21) Application number: 99974146.5
(22) Date of filing: 01.11.1999
(51) Int. Cl.: C07D 295/033, A61K 31/40, A61P 25/08

(54) **1-CYCLIC AMINO-ALKYLCYCLOHEXANE COMPOUNDS, PHARMACEUTICAL COMPOSITIONS THEREOF, AND THEIR USE AS ANTICONVULSANTS**
1-CYCLAMINO-ALKYLCYCLOHEXAN-DERIVATE, DIESE ENTHALTENDE PHARMAZEUTISCHE ZUSAMMENSETZUNGEN, UND IHRE VERWENDUNG ALS ANTICONVULSIA
COMPOSES AMINO-ALKYLCYCLOHEXANE 1-CYCLIQUES, LEURS COMPOSITIONS PHARMACEUTIQUES, ET LEUR UTILISATION COMME ANTICONVULSIVANTS

(43) Date of publication of application: 07.08.2002
(73) Proprietor: Merz Pharma GmbH & Co. KGaA, 60318 Frankfurt (DE)
(72) Inventor: GOLD, Markus, D-64569 Nauheim (DE); DANYSZ, Wojciech, D-61130 Nidderau (DE); PARSONS, Christopher, Graham, Raphael, D-61130 Nidderau (DE); KALVINSH, Ivars, LV-2169 Salaspils (LV); KAUSS, Valerjans, LV-1082 Riga (LV); JIRGENSONS, Aigars, LV-1013 Riga (LV)
(74) Representative: Marsden, John Christopher
(86) International application number: PCT/EP1999/008317
(87) International publication number: WO 2001/032640

(56) References cited:
- WO-A-99/01416
- US-A- 3 097 207
- US-A- 6 034 134

## Description

The present invention is directed to 1-cyclic amino-alkylcyclohexane compounds selected from the group consisting of those of the formula 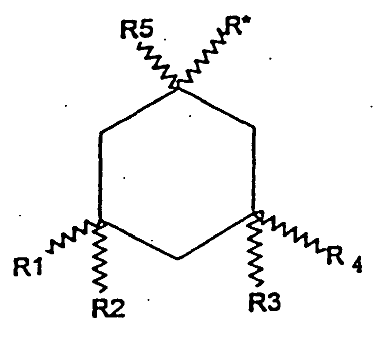
wherein R* is -(CH₂)ₙ-(CR⁶R⁷)ₘ-NR⁸R⁹
wherein n+m = 0, 1, or 2
wherein R¹ through R⁷ are independently selected from the group consisting of hydrogen and lower-alkyl (1-6C), at least R¹, R⁴ and R⁵ being lower-alkyl, and
wherein R⁸ and R⁹ together represent lower-alkylene -(CH₂)ₓ-
wherein x is 2 to 5, inclusive, and enantiomers, optical isomers, hydrates, and pharmaceutically-acceptable salts thereof, as well as pharmaceutical compositions thereof, and the preparation and use thereof as anticonvulsants for the treatment of convulsions or seizures in a living animal.

Of particular interest are compounds of the foregoing formula wherein R¹ through R⁵ are methyl, those wherein x is 4 or 5, and in particular the compound N-(1,3,3,5,5-pentamethylcyclohexyl) pyrrolidine, and optical isomers, enantiomers, hydrates and pharmaceutically-acceptable salts thereof.

In our published application WO 99/01416, published January 14, 1999, we disclosed compounds of the foregoing formula but wherein R⁸ and R⁹ were selected from hydrogen and lower-alkyl (1-6C), pharmaceutical compositions thereof, and their use as NMDA-receptor antagonists. It has now been found that compounds of the foregoing formula wherein R⁸ and R⁹ together represent lower-alkylene-(CH₂)x-, wherein x is 2-5, inclusive, and especially N-(1,3,3,5,5-pentamethylcyclohexyl) pyrrolidine, and optical isomers, enantiomers, hydrates and pharmaceutically-acceptable salts thereof, in addition to their NMDA antagonist properties, quite unpredictably possess a high degree of anticonvulsant activity in the kindling model, whereas other NMDA antagonists despite even higher NMDA-antagonistic properties are not active.

### SUMMARY OF THE INVENTION

What we therefore believe to be comprised by our present invention may be summarized, inter alia, in the following words:
A 1-cyclic amino-alkylcyclohexane compound selected from the group consisting of those of the formula 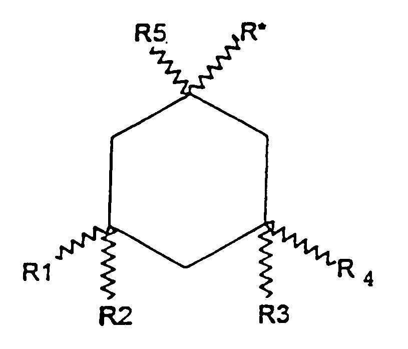
   wherein R* is -(CH₂)ₙ-(CR⁶R⁷)ₘ-NR⁸R⁹
   wherein n+m = 0, 1, or 2
   wherein R¹ through R⁷ are independently selected from the group consisting of hydrogen and lower-alkyl (1-6C), at least R¹, R⁴, and R⁵ being lower-alkyl, and wherein R⁸ and R⁹ together represent lower-alkylene -(CH₂)ₓ- wherein x is 2 to 5, inclusive, and enantiomers, optical isomers, hydrates, and pharmaceutically-acceptable salts thereof.
such a compound wherein R¹ through R⁵ are methyl;
such a compound wherein x is 4 or 5; and such a compound selected from the group consisting of
   N-(1,3,3,5,5-pentamethylcyclohexyl) pyrrolidine, and optical isomers, enantiomers, hydrates and pharmaceutically-acceptable salts thereof.

Also, a pharmaceutical composition comprising an effective amount of such compound in combination with one or more pharmaceutically-acceptable diluents, excipients, or carriers;
such a pharmaceutical composition wherein the effective amount is an effective anticonvulsant amount;
such a pharmaceutical composition wherein R¹ through R⁵ are methyl;
such a pharmaceutical composition wherein x is 4 or 5; and
such a pharmaceutical composition wherein the compound is selected from the group consisting of
N-(1,3,3,5,5-pentamethylcyclohexyl) pyrrolidine, and optical isomers, enantiomers, hydrates and pharmaceutically-acceptable salts thereof.

Further, the use of a 1-cyclic amino-alkylcyclohexane selected from the group consisting of those of the formula 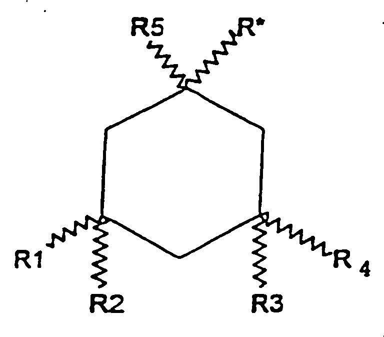
wherein R* is -(CH₂)ₙ-(CR⁶R⁷)ₘ-NR⁸R⁹
wherein n+m = 0, 1, or 2
wherein R¹ through R⁷ are independently selected from the group consisting of hydrogen and lower-alkyl (1-6C), at least R¹, R⁴ and R⁵ being lower-alkyl, and
wherein R⁸ and R⁹ together represent lower-alkylene-(CH₂)ₓ-
wherein x is 2 to 5, inclusive, and optical isomers, enantiomers, hydrates, and pharmaceutically-acceptable salts thereof, in the manufacture of a medicament to treat a living animal for alleviation of convulsions or seizures;
such a use wherein R¹ through R⁵ are methyl;
such a use wherein x is 4 or 5; and
such a use wherein the compound is selected from the group consisting of
   N-(1,3,3,5,5-pentamethylcyclohexyl) pyrrolidine, and optical isomers, enantiomers, hydrates and pharmaceutically-acceptable salts thereof.

Additionally, a method of making such a compound which comprises the step of reacting the corresponding 1-free amino-alkylcyclohexane with (1) an omega-haloalkylnitrile and cyclizing the resulting N-(omega-cyanoalkyl) compound to the corresponding 1-cyclic amino-alkylcyclohexane compound or with (2) an alpha, omega-dihaloalkyl compound.

### METHODS - CHEMISTRY

The starting materials for the preparation of the compounds of the present invention are known in the art. In our prior published application WO99/01416, PCT/EP98/04026, numerous 1-amino-alkylcyclohexylamine compounds are disclosed. Attention is called, for example, to Compound 5 on page 9 thereof and the numerous examples thereof throughout that publication. The starting compounds for the present invention have the formula set forth above but wherein both R⁸ and R⁹ are hydrogen. Of particular interest as a starting material is a compound selected from the group consisting of
1-amino-1,3,5-trimethylcyclohexane,
1-amino-1(trans),3(trans),5-trimethylcyclohexane,
1-amino-1(cis),3(cis),5-trimethylcyclohexane,
1-amino-1,3,3,5-tetramethylcyclohexane,
1-amino-1,3,3,5,5-pentamethylcyclohexane,
1-amino-1,3,5,5-tetramethyl-3-ethylcyclohexane,
1-amino-1,5,5-trimethyl-3,3-diethylcyclohexane,
1-amino-1,5,5-trimethyl-cis-3-ethylcyclohexane,
1-amino-(1S,5S)cis-3-ethyl-1,5,5-trimethylcyclohexane,
1-amino-1,5,5-trimethyl-trans-3-ethylcyclohexane,
1-amino-(1R,5S)trans-3-ethyl-1,5,5-trimethylcyclohexane,
1-amino-1-ethyl-3,3,5,5-tetramethylcyclohexane, and
1-amino-1-propyl-3,3,5,5-tetramethylcyclohexane, and pharmaceutically-acceptable acid addition salts of any of the foregoing. The compounds of the present invention are prepared therefrom with the additional step of converting the free amine compounds, preferably in the form of an acid addition salt such as the hydrochloride or the like, in a two-step cyclization procedure involving an intermediate N-cyanoalkyl compound and cyclization thereof to produce the desired 1-cyclic amino-alkylcyclohexane compound wherein the 1-amino group is in the form of a cyclic group, such as pyrrolidine or piperidine or the like, which compounds are the subject matter of the present invention.

### DETAILED DESCRIPTION OF THE INVENTION

The following further details and detailed examples are given by way of illustration only and are not to be construed as limiting.

### EXAMPLES

The 1-amino-alkylcyclohexane compounds, wherein the 1-amino group is cyclic, that is, wherein R⁸ and R⁹ together represent lower-alkylene -(CH2)x- wherein x is 2 to 5, inclusive, thereby presenting the 1-amino group -NR⁸ R⁹ in the form of a cyclic amine, are prepared in the following manner:

### Preparation of N-(3-Cyanopropyl)-1,3,3,5,5-pentamethylcyclohexylamine

A mixture of 1,3,3,5,5-pentamethylcyclohexylamine hydrochloride (2.06g, 10 mmol), 4-bromobutyronitrile (1.55g, 10.5 mmol) and sodium carbonate (3.18 g, 30 mmol) in tetrahydrofuran (50 ml) was refluxed for 85 h, then poured into water (100 ml) and extracted with ether (3*30 ml). The combined organic phases were washed with brine (20 ml) and dried over K₂CO₃. The solution was filtered and evaporated and the crude product was purified by chromatography on silica gel, eluting with hexane-ether (10:1), (6:1), (4:1) to give the product (1.86 g, 86%) as a colorless oil.
PMR spectrum: (CDCl₃, TMS) δ:0.87 (6H, s, c-Hex 3,5-CH₃); 1.06 (3H, s, c-Hex 1-CH₃; 1.18 (6H, s, 3,5-CH₃); 0.9-1.6 (7H, m, c-Hex ring protons and NH); 1.75 (2H, m, -CH₂-); 2.43 (2H, t, J=7Hz, CH₂N) and 2.66 ppm. (2H, t, J=7Hz, CH₂CN).

### Preparation of MRZ 2/705, namely: N-(1,3,3,5,5-Pentamethylcyclohexyl) pyrrolidine hydrochloride

N-(3-Cyanopropyl)-1,3,3,5,5-pentamethylcyclohexylamine (1.2 g, 5.1 mmol) in ethanol (120 ml) and conc. HCl (4 ml) was hydrogenated over 10% Pd/C (250 mg) at 7 bar for 40 h (after 24 h additional portion of catalyst (260 mg) was added). The catalyst was removed by filtration through celite pad and, solvent evaporated. The residue was treated with acetonitrile, the solids filtered off and the filtrate evaporated. The crude product was crystallized from ether to give N-(1,3,3,5,5-pentamethylcyclohexyl)pyrrolidine hydrochloride (0.67 g, 49%) with m.p. 156-158°C.
PMR spectrum: (DMSO-D₆, TMS) δ:0.97 (6H, s, 3,5-CH₃); 1.11 (6H, s, 3,5-CH₃); 0.8 - 1.4 (2H, cyclohexane 4-CH₂) 1.41 (3H, s, 1-CH₃); 1.69 (4H, m, cyclohexane 2,6-CH₂); 1.84 (4H, m, pyrrolidine 3,4-CH₂); 3.20 (4H, m, pyrrolidine 2,5-CH₂); 10.9 ppm (1H, br s, HN*). *Elemental analysis:* C₁₅H₂₉N*HCl*0.5H₂O):
Found (%) C 67.7; H 11.5; N 5.5
Calculated (%) C 67.0; H 11.6; N 5.2

Additional 1-cyclic amino compounds are prepared in the same manner starting from the selected alkyl-substituted cyclohexylamine, usually in the form of an acid addition salt such as the hydrochloride, and the selected ω-bromoalkylnitrile, such as 4-bromobutyronitrile, 3-bromopropionitrile, 2-bromoacetonitrile, and 5-bromovaleronitrile, in the manner of the preceding preparation, first to produce the selected N-ω-cyanoalkyl-alkylcyclohexylamine compound and then to cyclize the N-ω-cyanoalkyl-alkylcyclohexylamine compound into the resulting N-(alkylcyclohexyl) cyclic amine compound, namely, the pyrrolidine, piperidine, or other cyclic amine compound, wherein the nitrogen atom and R⁸ and R⁹ together form the cyclic amine moiety, R⁸ and R⁹ together representing a lower-alkylene chain of the formula -(CH₂)ₓ- wherein x is 2 to 5, inclusive.

Thus N-(1,3,3,5,5-pentamethylcyclohexyl) piperidine hydrochloride or other acid addition salt and numerous other lower-alkyl substituted cyclohexanes having a 1-pyrrolidino or 1-piperidino group or other 1-cyclic amino group are prepared according to the invention, depending upon the ω-bromoalkylnitrile and the alkyl-substituted cyclohexylamine starting materials selected for the reaction.

The method involved can therefore be appropriately described as the step of reacting the corresponding 1-free amino-alkylcyclohexane with an omega-haloalkylnitrile and cyclizing the resulting N-(omega-cyanoalkyl) compound to the corresponding 1-cyclic amino-alkylcyclohexane compound. The halogen is preferably bromine and the starting amine is preferably reacted in the form of an acid addition salt thereof such as the hydrochloride. Starting from the preferred starting materials as set forth in the foregoing, the following compounds are thus readily prepared:
N-(1,3,5-trimethylcyclohexyl)pyrrolidine or piperidine,
N-[1(trans),3(trans),5-trimethylcyclohexyl]pyrrolidine or piperidine,
N-[1(cis),3(cis),5-trimethylcyclohexyl]pyrrolidine or piperidine,
N-(1,3,3,5-tetramethylcyclohexyl)pyrrolidine or piperidine,
N-(1,3,3,5,5-pentamethylcyclohexyl)pyrrolidine or piperidine,
N-(1,3,5,5-tetramethyl-3-ethylcyclohexyl)pyrrolidine or piperidine,
N-(1,5,5-trimethyl-3,3-diethylcyclohexyl)pyrrolidine or piperidine,
N-(1,5,5-trimethyl-cis-3-ethylcyclohexyl)pyrrolidine or piperidine,
N-[(1S,5S)cis-3-ethyl-1,5,5-trimethylcyclohexyl]pyrrolidine or piperidine,
N-(1,5,5-trimethyl-trans-3-ethylcyclohexyl)pyrrolidine or piperidine,
N-[(1R,5S)trans-3-ethyl-1,5,5-trimethylcyclohexyl]pyrrolidine or piperidine,
N-(1-ethyl-3,3,5,5-tetramethylcyclohexyl)pyrrolidine or piperidine, and
N-(1-propyl-3,3,5,5-tetramethylcyclohexyl)pyrrolidine or piperidine, and pharmaceutically-acceptable salts of any of the foregoing.

R¹ through R⁷ may thus be, for example, methyl, ethyl, propyl, or a combination of these lower-alkyl groups and, as previously stated, at least R¹, R⁴, and R⁵ are preferably one of these groups and, additionally, most preferred compounds have R¹ through R⁵ as methyl.

### Alternative Procedure

The same compounds may be prepared by reacting the corresponding 1-free amino-alkylcyclohexane and the selected alpha, omega-dihaloalkyl compound, e.g., 1,3-dibromopropane, 1,4-dibromobutane, or 1,5-dibromopentane, according to the following representative example:

### N-(1,3,3,5,5-pentamethylcyclohexyl)pyrrolidine hydrochloride

1,3,3,5,5-pentamethylcyclohexylamine hydrochloride (12 g, 58.3 mmol), potassium carbonate (48.4 g, 350 mmol) and 1,4-dibromobutane (7.32 ml, 61.3 mmol) were refluxed in acetonitrile (250 ml) for 60h. After cooling to r.t., the mixture was filtered and the precipitate was washed with diethyl ether (600 ml). The filtrate was concentrated in vacuo by rotary evaporation and the residue was fractionally distilled at reduced pressure (11mm/Hg). The fraction at 129°C was collected to obtain colorless oil (8.95 g). This was dissolved in diethyl ether (120 ml) and 2.7 M HCl solution in diethyl ether (30 ml) was added. The resulting precipitate was filtered off, washed with diethyl ether (3*30 ml) and dried in vacuo over NaOH to give N-(1,3,3,5,5-pentamethylcyclohexyl)pyrrolidine hydrochloride hydrate (12.9 g, 68%) with m.p. 158°C. PMR spectrum: (DMSO-d6, TMS) d: 0.97 (6H, s, 3,5-CH3); 1.11 (6H,s, 3,5-CH3); 0.8 - 1.4 (2H, cyclohexane 4-CH2) 1.41 (3H, s, 1-CH3); 1.69 (4H, m, cyclohexane 2,6-CH2); 1.84 (4H, m, pyrrolidine 3,4-CH2); 3.20 (4H, m, pyrrolidine 2,5-CH2); 10.9 ppm (1H, br s, NH+).
*Elemental analysis* (C15H29n*HCl*H2O) Found (%) C 65.0; H 11.7; N5.0 Calculated (%) C 64.8; H 11.6; N 5.0.

### PHARMACOLOGICAL RESULTS

A. The following Tables 1 and 2 present pharmacological results with the compound MRZ 2/705 of the present invention. The Tables show the following:

**TABLE 1**

| MRZ 2/ | MES ED₅₀ mg/kg i.p. | | TI Tract | TI Rot. | Min I.ct-hality mg/kg i.p. |
|---|---|---|---|---|---|
| 705 | 9.55 | (4.3 - 21.1) | 3.9 | 5.4 | >50 |

### TABLE 1

Effect of MRZ 2/705 on convulsions induced by maximal electroshock (MES). Values are ED₅₀s in mg/kg (95% confidence limits are shown in parentheses). The therapeutic index (TI) was also calculated as the ED₅₀ for inhibition of traction reflex (Tract.) impairment or rotarod failure (Rot.) divided by the ED₅₀ for MES-induced seizure-induced convulsions.

**TABLE 2**

| MRZ | MK-801 Ki (µM) | SEM | Patch Clamp IC₅₀ (µM) | SEM | Glut Tox IC₅₀ (µM) | SEM |
|---|---|---|---|---|---|---|
| 705 | 7.14 | 1.7 | 25.40 | 4.1 | 12.32 | 1.19 |

### TABLE 2

Effect of MRZ 2/705 on [³H]-(+)-MK-801 binding, NMDA-induced currents in patch clamp experiments, and glutamate toxicity in cultured cortical neurones. Binding Ki values are means ±SEM of 3-5 experiments and were determined according to the Cheng-Prusoff relationship with a Kd for MK-801 of 4.6 nM. IC₅₀s (±SEM) in patch clamp and glutamate toxicity experiments were determined from data from at least 3 concentrations producing between 15 and 85% inhibition and at least 5 cells/well per concentration.

### B. Anticonvulsant activity of MRZ 2/705 in the amygdala kindling model

### Introduction

The substance MRZ 2/705 was tested in the amygdala kindling model in rats. In this model chronically implanted animals with stimulation and recording electrodes in the amygdala were rendered more susceptible to seizures by repeated initially subconvulsive electrical stimulation (Goddard et al., 1969, Sato el. al, 1990). Once enhanced sensitivity, characterized by generalized motor seizures, has developed, the animal is said to be fully kindled. For substance screening procedures we determine the individual threshold current that induces afterdischarges at the stimulation site.

### Materials and Methods

### Animals

Female Wistar rats were purchased at a body weight of 200-220g (Harlan Windelmann Versuchstierzucht, Borchen, Germany) and were then kept under controlled environmental conditions (24-25°C, 50-60% humidity, 12h light / dark cycle) with free access to standard laboratory chow (Altromin 1324 standard diet) and tap water. All experiments were performed within the same day time in the morning to minimize possible effects of circadian variation. During the period of experiments, animals had a body weight between 265 and 414g. These animals were previously kindled and used to test other compounds. The period between the previous and current studies was long enough (at least one month) so that it was ample time for a complete washout from the previously tested drug.

### Electrode implantation

For implantation of kindling electrodes, rats were anaesthetized with chloral hydrate (360 mg/kg, i/p.), the skull surface was exposed, and a bipolar electrode was implanted into the right hemisphere aimed at the basolateral amygdala using the following sterotaxic coordinates according to the atlas of Paximos and Watson (1986): 2.2 mm caudal, 4.8 mm lateral, 8.5 mm ventral (all respective to bregma). The electrodes consisted of two twisted Teflon-coated stainless steel wires (250 µm diameter) separated by 0.5 mm at the tip. A screw, which served as grounding electrode, was positioned over the left parietal cortex. Bipolar and ground electrodes were connected to plugs, and the electrode assembly and anchor screws were held in place with dental carylic cement applied to the exposed skull surface. After surgery, the rats were treated with antibiotics for 1 week to prevent infection.

**Kindling procedure and experiments in fully kindled animals**

Following a post-operative recovery period of two weeks, constant current stimulations (500µA, 1 msec, monophasic square-wave pulses, 50 Hz for 1 s) were delivered to the amygdala once daily (five times per week) until at least 10 sequential fully kindled stage-5 seizures were elicited. Seizure severity (SS) was scored according to Racine (1972): 1 immobility, eye closure, ear twitching, twitching of vibrissae, sniffing, facial clonus; 2 head nodding associated with more severe facial clonus; 3 clonus of one forelimb; 3.5 bilateral clonus without rearing; 4 bilateral clonus accompanied by rearing; 4.5 generalized clonic seizures without rearing and falling (e.g. because of direct loss of balance); 5 rearing and falling accompanied by generalized clonic seizures. In these fully kindled rats afterdischarge threshold (ADT) was determined by administering a series of stimulations at intervals of 1 min. increasing in steps of about 20% of the previously applied current. The afterdischarge threshold was defined as the lowest current intensity producing afterdischarge with a duration of at least 5 s. Determination of afterdischarge threshold was repeated two times to prove reproducibility before animals were used for anticonvulsant drug testing.

In all experiments seizure duration and afterdischarge duration were recorded in addition to seizure severity and afterdischarge threshold. Seizure duration 1 (SD1) was the time period of limbic and/or motor seizures. Limbic seizure activity which sometimes occurred after termination of secondarily generalized seizures was not included in seizure duration. Seizure duration 2 (SD2) was the time period from stimulation until the end of postictal depression phase.

Afterdischarge duration 1 (ADD1) was defined as the period of high amplitude spiking (at least 1 Hz frequency and twice the prestimulation amplitude) in the electroencephalogram (EEG) of the BLA electrode, including the time of stimulation. If the first period of spiking was directly followed by spiking with different amplitude, the time from the stimulation until the end of high and low amplitude spiking was taken as afterdischarge duration 2 (ADD2). Secondary afterdischarges which sometimes occur after a period of silent EEG were in all protocols marked by an X.

### Drug experiments

The substance MRZ 2/705 was dissolved in distilled water and administered i.p. Thereby applicated volume was 3 ml/kg body weight. dosages used in the experiments were 5, 10, and 20 mg/kg.

Animals were allowed to adapt to the laboratory environment, then body temperature was measured and animals were put into open cages for constant observation. 15 and 30 minutes following drug or vehicle administration behavioral alterations and body temperature were determined. Adverse effects were scored during observation in open cages and in an open field. In addition, rats were subjected to the rotarod test (polypropylene, foam-coated rod, 5cm in diameter, 8rpm). Animals were considered to have failed this test, when they fell from the rod in each of three consecutive 1-min. attempts.

Side effects were scored as follows: ataxia: 0=absent, 1=slight ataxia in hindlegs, 2=more pronounced ataxia with dragging of hindlegs, 3=further increase of ataxia and more pronounced dragging of hindlegs, 4=marked ataxia and loss of balance during forward locomotion, 5=very marked ataxia with frequent loss of balance, and 6=permanent loss of righting reflex; sedation: 0=absent, 1=slightly reduced forward locomotion, 2=reduced locomotion with rest periods between periods of locomotion, 3=reduced locomotion with more frequent rest periods, and 4=no forward locomotion and animal sits quietly with closed eyes; further adverse effects: 0=absent, 1=equivocal, 2=present, and 3=intense.

Statistical significance of seizure data was calculated by Wilcoxon signed rank test for paired replicates.

### Results

The substance MRZ 2/705 showed a dose-dependent anticonvulsant effect in amygdala kindled rats.

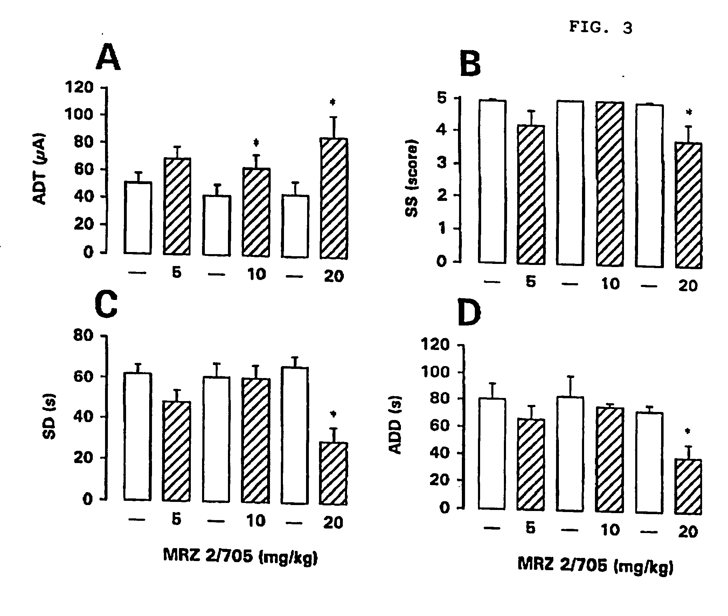

With the different dosages we found an average increase in afterdischarge threshold of 36% (5 mg/kg), 50% (10 mg/kg) and 95% (20 mg/kg) above control threshold. This increase proved to be significant following dosages of 10 and 20 mg/kg (Fig.3).

Besides elevation of the afterdischarge threshold the highest dosage tested (20 mg/kg) reduced seizure severity, duration of motor seizures, and duration of afterdischarges in a significant manner. With lower dosages these seizure parameters remained unchanged or were only influenced slightly.

With all three dosages we found minor signs of sedation. Slight ataxia was seen following dosages of 10 and 20 mg/kg, but no failure to pass the rotarod test was observed.

### Conclusions

In the present investigations the substance MRZ 2/705 potently increased the afterdischarge threshold in fully kindled rats. Furthermore with higher dosages the test substance significantly reduced seizure duration, afterdischarge duration and seizure severity recorded at ADT current. These results point to the fact that MRZ 2/705 possesses a potent anticonvulsant effect with inhibition of seizure initiation as well as seizure spread.

With regard to the predictability of the kindling model for drug efficacy against complex-partial seizures in humans (SATO et al., 1990; Löscher and Schmidt, 1994), our data predicts an anticonvulsant activity of MRZ 2/705 in temporal lobe epilepsy. Furthermore the substance did not induce severe behavioral adverse effects at the doses tested, and thus has a favorable profile with regard to side effects.

Figure 3 summarizes the kindling data of different doses of the test substance MRZ 2/705. Data are mean values ± SEM. Individual graphs give results on afterdischarge threshold (ADT), seizure severity (SS), seizure duration (SD), and afterdischarge duration (ADD).

### IN THE DRAWINGS:

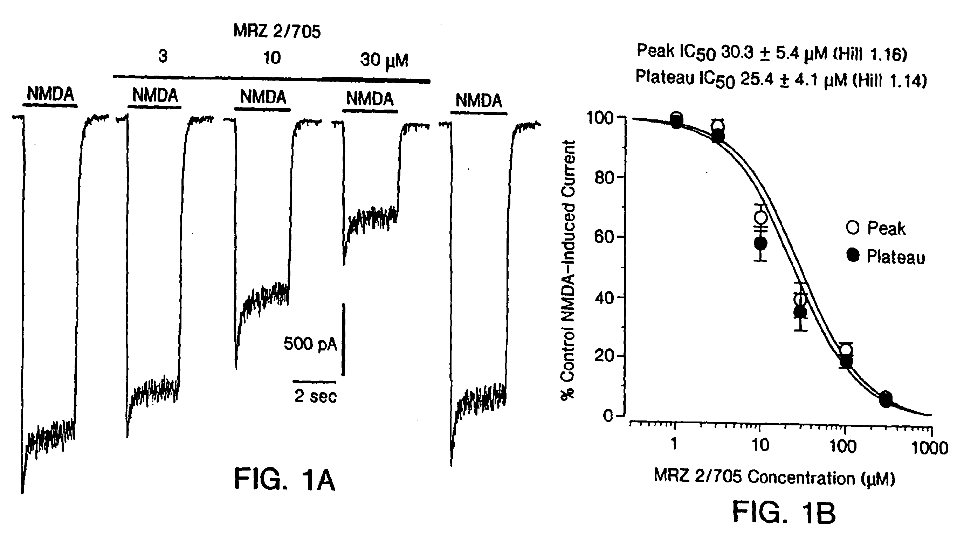

FIG. 1A and FIG. 1B show values obtained for the effects of Tables 1 and 2 using MRZ 2/705.

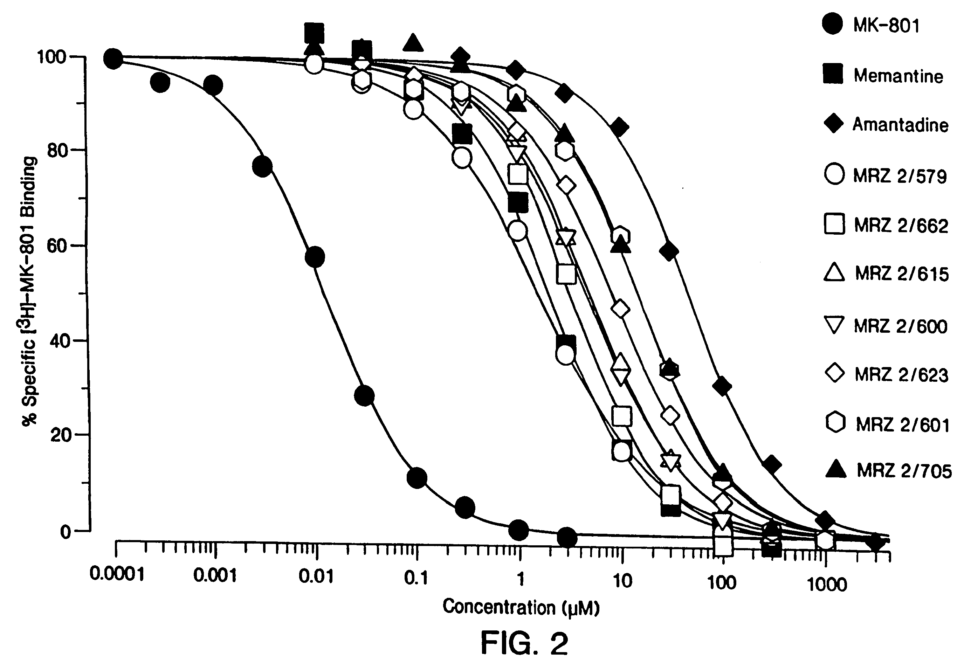

FIG. 2 shows values obtained for various closely-related but noncyclic amino compounds and MRZ 2/705 as well as reference standards in the specific [³H]-MK-801 binding test plotted against concentration.
FIG. 3 summarizes the kindling data of different doses of the test substance MRZ 2/705.

The compounds of the invention thus find application in the treatment of disorders of a living animal body, especially a human, in both NMDA and non-NMDA indications, in addition to their outstanding anticonvulsant and anti-seizure activity, especially as shown in the "kindling" model.

The method-of-treating a living animal body with a compound of the invention for the alleviation of the selected ailment therein, especially convulsions or seizures, is by any normally-accepted pharmaceutical route, employing the selected dosage which is effective in the alleviation of the particular ailment desired to be alleviated.

Use of the compounds of the invention in the manufacture of a medicament or the treatment of a living animal for alleviation of the selected ailment, especially convulsions or seizures, is carried out in the usual manner comprising the step of admixing an effective amount of a compound of the invention with a pharmaceutically-acceptable diluent, excipient, or carrier, and the method-of-treating, pharmaceutical compositions, and use of a compound of the present invention in the manufacture of a medicament are all in accord with the disclosure of our prior WO 99/01416 publication for the related 1-noncyclic amino compounds, and representative acid addition salts and their method of preparation are likewise disclosed in our prior published application for the corresponding 1-noncyclic amino-alkylcyclohexane compounds.

Representative pharmaceutical compositions prepared by admixing the active ingredient with a suitable pharmaceutically-acceptable excipient, diluent, or carrier, include tablets, capsules, solutions for injection, liquid oral formulations, aerosol formulations, TDS formulations, and nanoparticle formulations, thus to produce medicaments for oral, injectable, or dermal use, also in accord with the examples of pharmaceutical compositions given in our published application WO 99/01416 for the corresponding 1-noncyclic-amino-alkylcyclohexanes.

A typical pharmaceutical composition is thus manufactured by admixture of the following ingredients into a tablet formulation incorporating a 1-cyclic amino-cyclohexane active ingredient of the invention.

### Tablet Formulation

A suitable formulation for a tablet containing 10 milligrams of active ingredient is as follows:

| | Mg. |
|---|---|
| Active Ingredient | 10 |
| Lactose | 63 |
| Microcrystalline Cellulose | 21 |
| Talcum | 4 |
| Magnesium stearate | 1 |
| Colloidal silicon dioxide | 1 |

It is to be understood that the invention is not to be limited to the exact details of operation, or to the exact compositions, methods, procedures, or embodiments shown and described, as obvious modifications and equivalents will be apparent to one skilled in the art, and the invention is therefore to be limited only by the full scope which can be legally accorded to the appended claims.

### REFERENCES

Goddard, G.V., D.C. McIntyre, and C.K. Leech, 1969, "A permanent change in brain function resulting from daily stimulation", Exp. Neurol. 25:295-330.

Löscher, W. and D. Schmidt, 1994, "Strategies in antiepileptic drug development: Is rational drug design superior to random screening and structural variation?", Epilepsy Res. 17:95-134.

Racine, R.J., 1972, "Modification of seizure activity by electrical stimulation", II. Motor seizure Electroencephalograph. Clin. Neurophys. 32:295-299.

Sato, M., R.J. Racine, and D.C. McIntyre, 1990, "Kindling: Basic mechanisms and clinical validity", Electroencephalograph. Clin. Neurophys. 76:459-472.

See also: Ebert and Löscher, "Pathophysiology of the Kindling phenomenon: Implications for the development of new antiepileptic drugs", Neuroforum 3/99, p. 76.

## Claims

1. A 1-cyclic amino-alkylcyclohexane compound selected from the group consisting of those of the formula 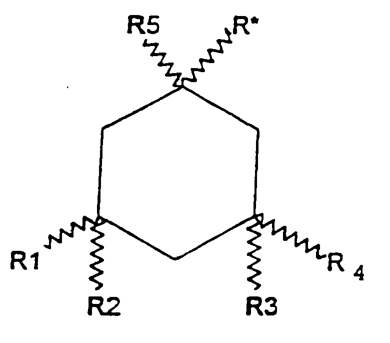
wherein R* is -(CH₂)ₙ-(CR⁶R⁷)ₘ-NR⁸R⁹
wherein n+m = 0, 1, or 2
wherein R¹ through R⁷ are independently selected from the group consisting of hydrogen and lower-alkyl (1-6C), at least R¹, R⁴, and R⁵ being lower-alkyl, and wherein R⁸ and R⁹ together represent lower-alkylene -(CH₂)ₓ- wherein x is 2 to 5, inclusive, and enantiomers, optical isomers, hydrates, and pharmaceutically-acceptable salts thereof.

2. A compound of Claim 1 wherein R¹ through R⁵ are methyl.

3. A compound of Claim 1 wherein x is 4 or 5.

4. A compound of Claim 2 wherein x is 4 or 5.

5. A compound of Claim 1 wherein the compound is selected from the group consisting of N-(1,3,3,5,5-pentamethylcyclohexyl) pyrrolidine, and optical isomers, enantiomers, hydrates and pharmaceutically-acceptable salts thereof.

6. A pharmaceutical composition comprising an effective amount of a compound of Claim 1 in combination with one or more pharmaceutically-acceptable diluents, excipients, or carriers.

7. A pharmaceutical composition of Claim 6 wherein the effective amount is an effective anticonvulsant amount.

8. A pharmaceutical composition of Claim 6 wherein R¹ through R⁵ are methyl.

9. A pharmaceutical composition of Claim 6 wherein x is 4 or 5.

10. A pharmaceutical composition of Claim 8 wherein x is 4 or 5.

11. A pharmaceutical composition of Claim 6 wherein the compound is selected from the group consisting of N-(1,3,3,5,5-pentamethylcyclohexyl) pyrrolidine, and optical isomers, enantiomers, hydrates and pharmaceutically-acceptable salts thereof.

12. Use of a 1-cyclic amino-alkylcyclohexane selected from the group consisting of those of the formula 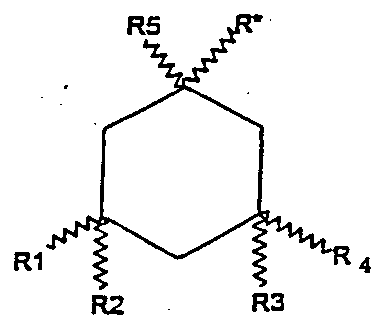
wherein R* is -(CH₂)ₙ-(CR⁶R⁷)ₘ-NR⁸R⁹
wherein n+m = 0, 1, or 2
wherein R¹ through R⁷ are independently selected from the group consisting of hydrogen and lower-alkyl (1-6C), at least R¹, R⁴ and R⁵ being lower-alkyl, and
wherein R⁸ and R⁹ together represent lower-alkylene -(CH₂)ₓ-
wherein x is 2 to 5, inclusive, and optical isomers, enantiomers, hydrates, and pharmaceutically-acceptable salts thereof, in the manufacture of a medicament to treat a living animal for alleviation of convulsions or seizures.

13. Use of Claim 12 wherein R¹ through R⁵ are methyl.

14. Use of Claim 12 wherein x is 4 or 5.

15. Use of Claim 13 wherein x is 4 or 5.

16. Use of Claim 12 wherein the compound is selected from the group consisting of N-(1,3,3,5,5-pentamethylcyclohexyl) pyrrolidine, and optical isomers, enantiomers, hydrates and pharmaceutically-acceptable salts thereof.

17. Method of making a compound of Claim 1 which comprises the step of reacting the corresponding 1-free amino-alkylcyclohexane with (1) an omega-haloalkylnitrile and cyclizing the resulting N-(omega-cyanoalkyl) compound to the corresponding 1-cyclic amino-alkylcyclohexane compound or with (2) an alpha, omega-dihaloalkyl compound.

## Patentansprüche

1. Eine 1-Cycloamino-alkylcyklohexan-Verbindung ausgewählt aus der Gruppe bestehend aus jenen der Formel 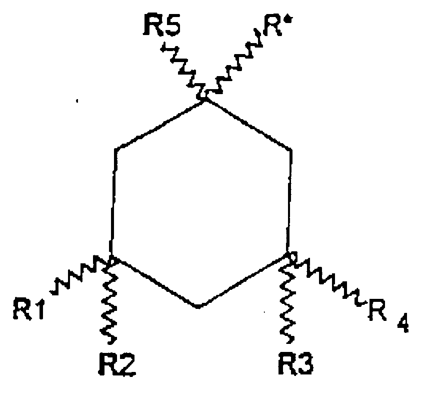
wobei R*-(CH₂)ₙ-(CR⁶R⁷)ₘ-NR⁸R⁹ ist,
wobei n + m = 0, 1 oder 2,
wobei R¹ bis R⁷ unabhängig voneinander ausgewählt werden aus der Gruppe bestehend aus Wasserstoff und Niederalkyl (1-6C), wobei mindestens R¹, R⁴ und R⁵ Niederalkyl sind und
wobei R⁸ und R⁹ Niederalken -(CH₂)ₓ- darstellen, wobei x von 2 bis einschließlich 5 sein kann, und Enantiomere, optische Isomere, Hydrate und pharmazeutisch verträgliche Salze davon.

2. Verbindung nach Anspruch 1, wobei R¹ bis R⁵ Methyl sind.

3. Verbindung nach Anspruch 1, wobei x 4 oder 5 ist.

4. Verbindung nach Anspruch 2, wobei x 4 oder 5 ist.

5. Verbindung nach Anspruch 1, wobei die Verbindung ausgewählt wird aus der Gruppe bestehend aus N-(1,3,3,5,5-Pentamethylcyklohexyl)-Pyrrolidin, und optischen Isomeren, Enantiomeren, Hydraten und pharmazeutisch verträglichen Salzen davon.

6. Pharmazeutische Zusammensetzung umfassend eine wirksame Menge einer Verbindung nach Anspruch 1 in Kombination mit einem oder mehreren pharmazeutisch verträglichen Verdünnungsmitteln, Exzipienten oder Trägem.

7. Pharmazeutische Zusammensetzung nach Anspruch 6, wobei die wirksame Menge eine wirksame krampflösende Menge ist.

8. Pharmazeutische Zusammensetzung nach Anspruch 6, wobei R¹ bis R⁵ Methyl sind.

9. Pharmazeutische Zusammensetzung nach Anspruch 6, wobei x 4 oder 5 ist.

10. Pharmazeutische Zusammensetzung nach Anspruch 8, wobei x 4 oder 5 ist.

11. Pharmazeutische Zusammensetzung nach Anspruch 6, wobei die Verbindung ausgewählt wird aus der Gruppe bestehend aus N-(1,3,3,5,5-Pentamethylzyklohexyl)-Pyrrolidin, und optischen Isomeren, Enantiomeren, Hydraten und pharmazeutisch verträglichen Salzen davon.

12. Verwendung eines 1-Cykloamino-alkylcyklohexans ausgewählt aus der Gruppe bestehend aus jener der Formel 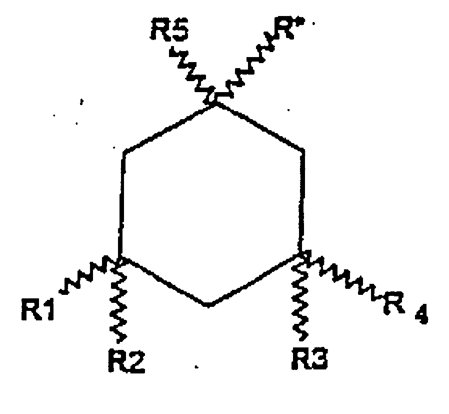
wobei R* -(CH₂)ₙ-(CR⁶R⁷)ₘ-NR⁸R⁹ ist,
wobei n + m = 0, 1 oder 2,
wobei R¹ bis R⁷ unabhängig voneinander ausgewählt werden aus der Gruppe bestehend aus Wasserstoff und Niederalkyl (1-6C), wobei mindestens R¹, R⁴ und R⁵ Niederalkyl sind, und
wobei R⁸ und R⁹ zusammen Niederalkylen -(CH₂)ₓ- darstellen, wobei x von 2 bis einschließlich 5 sein kann, und optische Isomere, Enantiomere, Hydrate und pharmazeutisch verträgliche Salze davon, für die Herstellung eines Medikaments zur Behandlung eines lebenden Tieres zur Linderung von Krämpfen oder Krampfanfällen.

13. Verwendung nach Anspruch 12, wobei R¹ bis R⁵ Methyl sind.

14. Verwendung nach Anspruch 12, wobei x 4 oder 5 ist.

15. Verwendung nach Anspruch 13, wobei x 4 oder 5 ist.

16. Verwendung nach Anspruch 12, wobei die Verbindung ausgewählt wird aus der Gruppe bestehend aus N-(1,3,3,5,5-Pentamethylcyklohexyl)-Pyrrolidin, und optischen Isomeren, Enantiomeren, Hydraten und pharmazeutisch verträglichen Salzen davon.

17. Verfahren zur Herstellung einer Verbindung nach Anspruch 1, wobei das Verfahren den Schritt des Reagierens des entsprechenden 1-freien Amino-alkylcyklohexans mit (1) einem omega-Haloalkylnitril und den Ringschluss der resultierenden N-(omega-Zyanoalkyl)-Verbindung zur entsprechenden 1-Cykloamino-alklyzyklohexan-Verbindung oder mit (2) einer alpha, omega-Dihaloalkyl-Verbindung, umfasst.

## Revendications

1. Composé 1-cyclique amino-alkycyclohexane sélectionné parmi le groupe consistant en ceux de formule 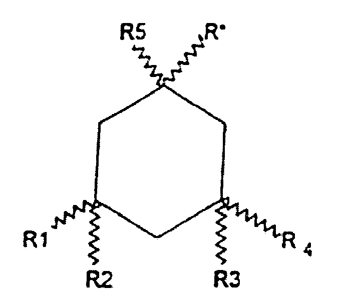 dans laquelle
R* est -(CH₂)ₙ-(CR⁶R⁷)ₘ-NR⁸R⁹
où n + m = 0, 1 ou 2
où R¹ à R⁷ sont indépendamment sélectionnés parmi le groupe consistant en un hydrogène et un alkyle inférieur (1-6C), au moins R¹, R⁴ et R⁵ étant des alkyles inférieurs et où R⁸ et R⁹ représentent ensembles un alkylène inférieur -(CH₂)ₓ- où x est de 2 à 5, inclus, ainsi que les énantiomères, les isomères optiques, les hydrates et les sels pharmaceutiquement acceptables de ceux-ci.

2. Composé selon la revendication 1, dans lequel R¹ à R⁵ sont des méthyles.

3. Composé selon la revendication 1, dans lequel x est 4 ou 5.

4. Composé selon la revendication 2, dans lequel x est 4 ou 5.

5. Composé selon la revendication 1, dans lequel le composé est sélectionné dans le groupe consistant en le N-(1,3,3,5,5-pentaméthylcyclohexyl) pyrrolidine, ainsi que les isomères optiques, les énantiomères, les hydrates et les sels pharmaceutiquement acceptables de celui-ci.

6. Composition pharmaceutique comprenant une quantité efficace d'un composé selon la revendication 1 en combinaison avec un ou plusieurs diluants, excipients, ou supports pharmaceutiquement acceptables.

7. Composition pharmaceutique selon la revendication 6, dans laquelle la quantité efficace est la quantité efficace anti-convulsivante.

8. Composition pharmaceutique selon la revendication 6, dans laquelle R¹ à R⁵ sont des méthyles.

9. Composition pharmaceutique selon la revendication 6, dans laquelle x est 4 ou 5.

10. Composition pharmaceutique selon la revendication 8, dans laquelle x est 4 ou 5.

11. Composition pharmaceutique selon la revendication 6, dans laquelle le composé est sélectionné dans le groupe consistant en le N-(1,3,3,5,5-pentaméthylcyclohexyl) pyrrolidine, ainsi que les isomères optiques, les énantiomères, les hydrates et les sels pharmaceutiquement acceptables de celui-ci.

12. Utilisation d'un 1-cyclique amino-alkycyclohexane sélectionné parmi le groupe consistant en ceux de formule 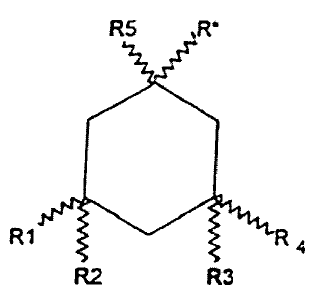
dans laquelle R* est -(CH₂)ₙ-(CR⁶R⁷)ₘ-NR⁸R⁹
où n+m=0, 1 ou 2
où R¹ à R⁷ sont indépendamment sélectionnés parmi le groupe consistant en un hydrogène et un alkyle inférieur (1-6C), au moins R¹, R⁴ et R⁵ étant des alkyles inférieurs et où R⁸ et R⁹ représentent ensembles un alkylène inférieur -(CH₂)ₓ- où x est de 2 à 5, inclus, ainsi que les énantiomères, les isomères optiques, les hydrates et les sels pharmaceutiquement acceptables de ceux-ci, pour la fabrication d'un médicament pour traiter un animal vivant pour atténuer des convulsions ou des attaques.

13. Utilisation selon la revendication 12, dans laquelle R¹ à R⁵ sont des méthyles.

14. Utilisation selon la revendication 12, dans laquelle x est 4 ou 5.

15. Utilisation selon la revendication 13, dans laquelle x est 4 ou 5.

16. Utilisation selon la revendication 12, dans laquelle le composé est sélectionné dans le groupe consistant en le N-(1,3,3,5,5-pentaméthylcyclohexyl) pyrrolidine, ainsi que les isomères optiques, les énantiomères, les hydrates et les sels pharmaceutiquement acceptables de celui-ci.

17. Méthode de préparation d'un composé selon la revendication 1 qui comprend l'étape de faire réagir le 1-libre amino-alkyl-cyclohexane correspondant avec (1) un oméga-haloalkylnitrile et de cyclisation du composé N(oméga-cyanoalkyl) résultant en le 1-cyclique amino-alkycyclohexane ou avec (2) un composé alpha,oméga-dihaloalkyl.
